# EUROPEAN PATENT APPLICATION

(11) **EP 1 642 598 A2**
(43) Date of publication of application: **05.04.2006**
(21) Application number: 05024340.1
(22) Date of filing: 02.04.2003
(51) Int. Cl.: A61L 2/14

(54) **System and method for injection of an organic based reagent into weakly ionized gas to generate chemically active species**

(30) Priority: 02.04.2002 US 369654 P
(62) Divisional of application: 03746142.3
(71) Applicant: Plasmasol Corporation, Hoboken, NJ 07030 (US)
(72) Inventor: Babko-Malyi, Sergei, West Windsor, NJ 08550 (US); Crowe, Richard, Hazlet, NJ 07730 (US)
(74) Representative: Held, Stephan

(57) **Abstract**

The present invention is directed to a method for increasing concentration of chemically active species generated by weakly ionized gas used to treat an object, said method comprising the steps of:
generating the weakly ionized gas using a reactor; and
introducing an organic based reagent in the presence of the weakly ionized gas to increase production of the chemically active species, wherein the organic based reagent includes ethylene and an oxidizer.

Further, a respective system is described.

## Description

### Cross-Reference to Related Applications

This application claims the benefit of U.S. Provisional Application No. 60/369,654, filed April 2, 2002, which is hereby incorporated by reference in its entirety.

### Field of the Invention

The present invention is directed to the generation of active species, and, in particular, to a system and method for the injection of an organic based reagent into weakly ionized gas to generate chemically active species, for example, ions and free radicals.

### Background of the Invention

Ethylene Oxide (EO) has been widely used since the 1950s as a low temperature sterilant for sterilizing temperature and moisture sensitive medical devices and supplies. It is believed that sterilization is achieved using EO through the mechanism referred to as "alkylation". During alkylation an ethyl group substitutes for hydrogen atoms on molecules needed to sustain life. This substitution prohibits normal life-supporting functions of such molecules as proteins and DNA. During low temperature sterilization processing a sufficient amount of EO is used to disrupt or prohibit microbial life.

Heretofore, EO sterilizers were typically combined with a chlorofluorocarbon sterilizing agent (e.g., CFC-12). In 1995 chlorofluorocarbon sterilizing agents were phased out due to environmental regulations regarding their destruction of the earth's ozone layer. The use of EO, however, still remains prevalent in sterilization of medical instruments due to its ability to disrupt microbial life in difficult to reach places; its wide variety of material compatibility and penetrability; and the economic constraints associated with replacing existing systems. Despite such benefits, EO as a sterilization agent has numerous disadvantages including: (i) the need to aerate after exposure to EO for patient and worker safety; (ii) relatively long cycle times to ensure quality sterilization and provide safe aeration; (iii) high toxicity and reactive hazards associated with EO; and (iv) high flammability associated with pure EO.

Environmental regulations have left a void in the sterilization industry that is exacerbated by the increased use of materials and instruments needed to be sterilized that are sensitive to heat and other chemical processes associated with alternative sterilization methods. Attention has focused on providing an alternative sterilizing agent that eliminates the use of EO but provides the same efficacy results. Numerous alternative sterilization methods have been developed to fulfill the needs of the medical industry such as using: (i) pure EO (which is highly flammable); (ii) a non-flammable EO/HCFC mixture; and/or (iii) a non-thermal plasma.

In particular, Johnson & Johnson has developed the Sterrad® low temperature gas sterilizing process using barrier discharge plasma technology. The principle mechanism is the use of the super-oxidizer, hydrogen peroxide. Although oxidants are more chemically reactive than alkylating sterilants (e.g., EO) making them more effective at low temperatures, oxidants disadvantageously are less likely to penetrate all portions of the objects being sterilized (especially areas such as long narrow lumens). Super-oxidizers destroy life-supporting molecules without requiring large quantities. Since its approval by the Food and Drug Administration as a sterilizer, the Sterrad® device has grown in acceptance by medical institutions. Nevertheless, the Sterrad® device is not suitable for use with packaging or other items that contain cellulose that will absorb the sterilant. Additional disadvantages of the Sterrad® device include: (i) load restraints due to the need to operate in a vacuum chamber; (ii) its inability to penetrate lumen; (iii) relatively high capital and operating costs; and (iv) relatively slow cycle times.

It is therefore desirable to develop an enhanced system and method that provides the same level of efficacy without all the drawbacks associated with the use of EO. Furthermore, it is desirable to develop a system and method for the treatment of solids, liquids, gases, vapors or any combination thereof that contain undesirable chemical and/or biological contaminants.

### Summary of the lnvention

The present invention is directed to a method for treatment of an object to destroy unwanted chemical and/or biological contaminants that overcomes the deficiencies associated with conventional methods.

Specifically, the invention is directed to a system and method for the injection of an organic based reagent into weakly ionized gas to generate chemically active species, e.g., ions and free radicals. The organic based reagent may be a combination of an organic additive (e.g., an alcohol (such as a C₁-C₅ alcohol or preferably a C₁-C₃ alcohol) or a C₂-C₆ alkene (such as ethylene)) mixed with an oxidizer (e.g., oxygen) prior to being introduced in the weakly ionized gas. Alternatively, the organic based reagent may be the injection of an organic additive alone in the weakly ionized gas while in the presence of air (non vacuum chamber) that inherently contains oxygen and serves as the oxidizer. Also, the organic based reagent may comprise an organic additive that itself includes an oxidizing component such as ethanol. In this situation the oxidizing component of the organic component when injected into the weakly ionized gas forms hydroxyl radicals, atomic oxygen or other oxidizing species that may be sufficient to eliminate the need for a supplemental oxidizer. Regardless of the organic based reagent used, the organic additive reacts with the oxidizer while in the presence of weakly ionized gas to initiate the production of chemically active species. This system and method may be used for sterilization whereby the increased concentrations of generated chemically active species accelerate and improve overall destruction rates of undesirable chemical and/or biological contaminants on surfaces and/or in fluid streams.

In addition, the invention relates to a method for increasing the concentration of chemically active species generated by weakly ionized gas used to treat an object. The weakly ionized gas is generated using a reactor. An organic based reagent, including an organic additive and an oxidizer, is introduced in the presence of the weakly ionized gas to increase production of the chemically active species. The object to be treated is exposed to the generated chemically active species to destroy undesirable chemical and/or biological contaminants.

Furthermore, the present invention is also directed to a system for carrying out the method described above.

### Brief Description of the Drawing

The foregoing and other features of the present invention will be more readily apparent from the following detailed description and drawings of illustrative embodiments of the invention wherein like reference numbers refer to similar elements throughout the several views and in which:
Figure 1 is a schematic diagram of a reactor system wherein an organic based reagent is injected into weakly ionized gas to generate chemically active species in accordance with the present invention;
Figure 2a is a cross-sectional view of an exemplary capillary discharge reactor wherein an organic based reagent is injected into weakly ionized gas to generate chemically active species in accordance with the present invention;
Figure 2b is a cross-sectional view of an exemplary capillary discharge reactor with auxiliary channels that are not in fluid communication with the capillary and wherein an organic based reagent is injected into weakly ionized gas to generate chemically active species in accordance with the present invention;
Figure 2c is a cross-sectional view of an exemplary capillary discharge reactor with auxiliary channels in fluid communication with the capillary and wherein an organic based reagent is injected into weakly ionized gas to generate chemically active species in accordance with the present invention;
Figure 3a is a partial longitudinal cross-sectional view of an exemplary slot discharge reactor wherein an organic based reagent is injected into weakly ionized gas to generate chemically active species in accordance with the present invention;
Figure 3b is a top view of the slot discharge reactor of Figure 3a;
Figure 4a is a perspective view of an exemplary annular slit discharge reactor with slits defined longitudinally in the first electrode and wherein an organic based reagent is injected into weakly ionized gas to generate chemically active species in accordance with the present invention;
Figure 4b is a perspective view of an alternative configuration of the inner first dielectric of Figure 4a wherein the slits are defined radially about the first electrode; and
Figure 4c is a perspective view with a cut away portion of yet another exemplary embodiment of a slit discharge reactor wherein slits are formed between adjacent rods positioned around an inner tube.

### Detailed Description of the Invention

The present inventive method utilizes organic additives (e.g., liquids, gases and/or vapors) injected into generated weakly ionized gas, preferably but not necessarily at atmospheric pressure, to produce chemically active species (e.g., ions and free radicals) that accelerate and improve overall destruction rates of undesirable chemical and/or biological contaminants on surfaces and/or in fluid streams (e.g., liquids, gases, vapors, or any combination thereof). Any reactor configuration may be employed that generates "weakly ionized gas" defined as gas discharge plasma or non-plasma media (mixture of charge and neutral particles) such that the ratio of concentrations (by volume) of neutral particles to that of charged particles is more than approximately 1000 to 1. The weakly ionized gas can, but need not necessarily be, "plasma" defined as a gas that contains positive, negative and neutral particles whose number density of negative charges is approximately equal of a number density of positive charges in a given volume. These reactors are energized by power sources such as direct current (DC), alternating current (AC) or radio frequency (RF) power supplies. Several exemplary reactor configurations are described in detail, however other configurations are contemplated and within the intended scope of this invention.

The reactor is configured so that an object to be treated (e.g., a solid or a fluid such as a liquid, vapor, gas, or any combination thereof), generally contaminated with undesirable chemicals (e.g., an atomic element or a compound) and/or biological agents (e.g., protein or DNA) is exposed to generated weakly ionized gas in which various processes such as oxidation, reduction, ion induced reactions, and/or electron induced reactions, efficiently allow for selective chemical processes or reaction mechanisms to occur. An organic based reagent is injected into the generated weakly ionized gas to enhance the output of one or more of these chemical processes. The organic based reagent is typically the combination of an organic additive (e.g., an alcohol, ethyl alcohol or ethylene) mixed with an oxidizer (e.g., oxygen) prior to being injected into the weakly ionized gas. Alternatively, the organic based reagent may be an organic additive introduced into the weakly ionized gas while in the presence of air (non vacuum chamber) that inherently contains oxygen. It is also within the intended scope of the invention to use an organic based reagent containing an organic additive that itself includes an oxidizing component such as EO or ethanol. In this situation the oxidizing component of the organic component when injected into the weakly ionized gas forms hydroxyl radicals, atomic oxygen or other oxidizing species. These generated oxidizing species may be sufficient to eliminate the need for a separate or supplemental oxidizer. However, if additional oxidation is desired, a supplemental oxidizer (e.g., oxygen) can be injected in the weakly ionized gas simultaneously with or after injection of the organic additive containing an oxidizing component to increase the concentration of oxidizing species thereby enhancing overall performance and efficiency. In any scenario, the organic vapors react with the oxidizer (from the air, as a supplemental source and/or as a component of the organic additive) while in the presence of weakly ionized gas to initiate the production of chemically active species, e.g., ions and free radicals. Selection of an organic additive to be injected into the weakly ionized gas can be used to promote or initiate specific chemical reactions with little, if any, heating up of the bulk gas.

By way of example, the present invention will be described with respect to the application of organic additives injected into weakly ionized gas to sterilize contaminated surfaces, liquid, gases, vapors and/or air streams. It is, however, within the intended scope of the invention to use this system and method for applications other than sterilization.

In the hereinafter described embodiments the dimensions of the reactor for generating weakly ionized gas may be selected, as desired, such that the residence time exposure of the pollutants to the generated active species is sufficient to ensure destruction of the undesirable chemical and/or biological contaminants to the desired level, for example, at the molecular level. The organic additive may be injected directly into the reactor in which the weakly ionized gas is generated. Selection of the size or dimension of the weakly ionized gas reactor may be chosen to generate selective chemical reactions using organic additives such that the lifetime of the active species generated by the weakly ionized gas extends beyond the weakly ionized gas generation region for a duration sufficient to effect a sterilization or oxidative process downstream of the reactor (e.g., so that the generated active species during their life cycle react with and destroy contaminants on a filter surface disposed downstream of the reactor).

Below are four exemplary reaction mechanisms that exist in weakly ionized gas enhanced chemistry responsible for the formation of the chemically active species. Common to all mechanisms are electron impact dissociation and ionization to form reactive radicals. The injection of an organic based reagent into the weakly ionized gas promotes one or more of these chemical reactions increasing the concentration of some of the generated chemically active species thereby improving the sterilization and decontamination process. Injection of an organic based reagent into the weakly ionized gas produces one or more of the following reaction chains that generate the chemically active species:

### 1. Formation of ions and ion clusters:

e + N₂ → N₂⁺ +2e e + O₂ → O₂⁺ + 2e

N₂⁺ + N₂ → N₄⁺ O₂⁺ + O₂ → O₄⁺

N₄⁺, N₂⁺ + O₂ → O₂⁺ + products

O₂⁺,Oₙ⁺ + H₂O → O₂⁺(H₂O)

O₂⁺( H₂O)+ H₂O → O₂⁺(H₂O)₂ → H₃O⁺(OH) + O₂

H₃O⁺(OH)+ H₂O→H₃O⁺(H₂O)+OH·

H₃O⁺(H₂O) + nH₂O → H₃O⁺(H₂O)₂ + (n-1)H₂O → H₃O⁺(H₂O)ₕ + (n-h)H₂O

Hydronium ion clusters can protonate ethyl alcohol when it is present in the organic based reagent:

H₃O⁺(H₂O)ₕ+ EtOH → EtOH₂⁺(H₂O)_{b} + (h+1-b)H₂O

Ion clusters such as EtOH₂⁺(H₂O)_{b} have a relatively long lifetime capable of surviving the transport to the surfaces of an object targeted for sterilization and provide an Et group for replacement of a hydrogen atom in bacterial DNA thereby killing targeted micro-organisms. Other organic ions, for example, ·C₂H₄OH⁺, ·C₂H₃OH⁺, ·CH₂OH⁺, ·CHOH⁺. CH₃OH⁺, ·C₂H₅⁺ may be formed and clustered. The role of each of these organic ions in the sterilization process depends on their lifetime and chemical activity.

### 2. Formation of free radicals:

e⁻ + O₂ → e⁻ + O + O(1D)

e⁻+ O₂ → e⁻ + O₂^{*}

e⁻ + N₂ → e⁻+N+N, N + O₂ → NO+O

e⁻ + N₂ → N₂^{*} + e⁻, N₂^{*}+ O₂ → N₂ + O + O

O + O₂ + M → O₃ + M, O₂^{*} + O₂ → O₃ + O

O(1D) + H₂O → 2 OH·

Other numerous reactions leading to formation of NO₂, HO₂· and other active species , for example, H₂O₂, are possible.

In the presence of organics, formation of organic radicals will occur:

RH + OH· → R· + H₂O, R· + O₂ + M → RO₂· + M,

RO₂· + NO → RO· + NO₂, RO· + NO₂ + M → RONO₂ + M,

RO· + O₂ → RCHO + HO₂·,

Organics and oxygen injected into the weakly ionized gas also promotes the formation of other organic radicals such as peroxy (RO₂), alkoxy (RO), acyl peroxyacyl (RC(O)OO), and byproducts, such as hydroperoxides (ROOH), peroxynitrates (RO₂NO₂), organic nitrates (RONO₂), peroxyacids (RC(O)OOH), carboxylic acids (RC(O)OH), peroxyacyl nitrates (RC(O)O₂NO₂), and other oxygen containing organic radicals. A predetermined concentration of each of these organic radicals will be produced regardless of which organic additive is used, the only difference being the concentration of each organic radical differs based on the organic additive employed. Preliminary experimental results seem to suggest that the most efficient sterilization is achieved using ethylene, however, other organic additive may be used to form partially oxidized products including alcohols. Thus, sterilization methods may be enhanced in the presence of an organic based reagent injected into the weakly ionized gas.

Heretofore, prior art sterilization methods relied on oxygen, hydrogen or nitrogen based active based species formed in the weakly ionized gas or on direct effects of electric fields, plasma or radiation. In contrast, the present inventive sterilization method relies on the chemically active species generated in response to the use of an organic based regent injected into the weakly ionized gas. Active species based on O, H, N atoms (e.g., NO₂., H₂O₂, O₃ and correspondent radicals such as HO₂·, OH·) are significantly less effective sterilizers than the chemically active species generated in response to the use of an organic based regent injected into the weakly ionized gas.

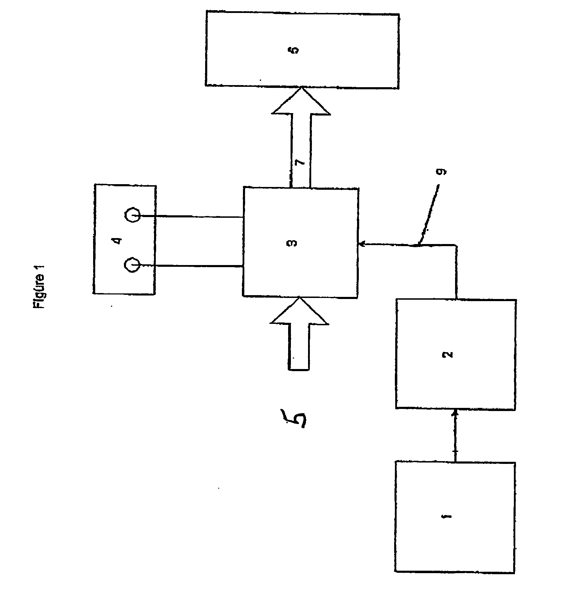

Figure 1 is a generic schematic diagram of the reactor system with injection of an organic based regent into the weakly ionized gas to generate chemically active species in accordance with the present invention. An oxidizer 1 (e.g., air or oxygen) is mixed with an organic additive 2 (e.g., ethylene, alcohol) to form an organic based reagent 9. By way of example, the organic based reagent 9 may be ethylene mixed with oxygen, or ethanol in the presence of air. Instead of introducing the oxidizer independently of that of the organic additive, the organic additive itself may include an oxygen component. For instance, EO or ethanol may be used as the organic additive and injected into the weakly ionized gas thereby producing hydroxyl radicals, atomic oxygen or other oxidizing species that serve as the oxidizer. These hydroxyl radicals may eliminate the need to independently introduce an oxidizer. To enhance performance, additional oxidizer may still, but need not be, injected independently into the weakly ionized gas even when using an organic based reagent containing an oxygen component.

The organic based reagent 9 may be a liquid, gas, vapor or any combination thereof. In addition, the organic based reagent 9 is introduced into the weakly ionized gas reactor 3, preferably, through an opening defined in a primary or first electrode, as described in detail further below, where the highest concentration of sterilizing species including chemically active species are generated.

A power supply 4 such as DC, AC, high frequency, RF, microwave, or pulsed, depending on the particular plasma source design, energizes the reactor 3. The object to be treated 5, typically a fluid to be treated such as a liquid, gas or vapor, may be passed together with the organic based reagent 9 into or proximate a region of the plasma reactor 3 in which the weakly ionized gas is generated. This is advantageous in that the object to be treated 5 is exposed to a high concentration of generated chemically active species present in the region in which the weakly ionized gas is generated. Alternatively, or in addition thereto, an object to be treated 5' (generally a solid object) may be disposed in the after glow beyond (e.g., downstream of) the region in which the weakly ionized gas is generated yet still subject to exposure to the chemically active species during their life span that were generated by the weakly ionized gas injected with the organic based reagent. In one particular example, the object to be treated 5' may be a particulate filter and/or contaminated object (e.g., contaminated solid surface, contaminated liquid, contaminated gas, or any combination thereof) housed within a sterilization chamber and subject to exposure by the sterilizing active species generated by the weakly ionized gas and injected organic based reagent. The objects to be treated 5, 5' may, but need not be the same. For example, a gas to be treated as an object 5 to be passed through the capillary where it is exposed to the chemically active species generated therein while a surface of a solid object 5' to be treated may be placed so as to be subject to direct exposure in the after flow of the generated chemically active species.

Sterilizing species generated by the weakly ionized gas and injected organic based reagent interact with chemical or biological (e.g., DNA and other building blocks of microorganisms) contaminants deposited on or contained within the object to be treated 5, 5'. For example, replacement of a hydrogen atom in bacterial DNA by an alkyl group (Cₙ H₂ₙ₊₁) due to exposure to the generated chemically active species leads to inactivation or destruction of microorganisms. Alkylation is believed to be responsible for sterilization in accordance with the inventive method described herein, however other mechanisms and chemically active species may be present. The present inventive sterilization system and method improves the overall efficacy while reducing the health and environmental hazards associated with conventional toxic sterilizing agents by employing biologically active yet relatively short living chemically active species generated by weakly ionized gas in the presence of an organic additive.

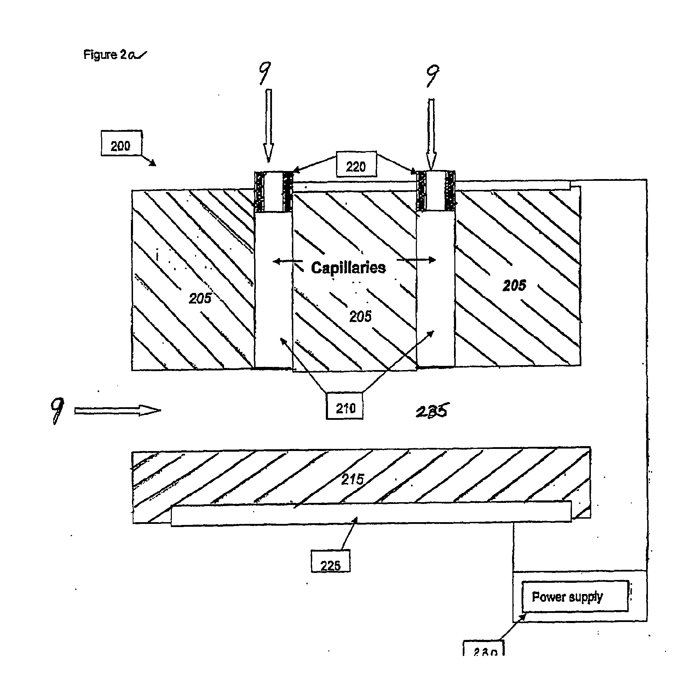

Numerous configurations of the weakly ionized gas generation reactor 3 are contemplated. Several exemplary non-thermal plasma reactor configurations at atmospheric pressure will be shown and described in detail, however, other configurations may be used, as desired. Figure 2a is a cross-sectional view of an exemplary capillary discharge reactor, as described in U.S. Patent Application Serial No. 09/738,923, filed on December 15, 2000, by the same assignees of the present invention. For illustrative purposes only, reactor 200 is shown having a first dielectric 205 with two capillaries 210 defined therethrough and a second dielectric 215 separated from the first dielectric 205 to form a channel 235 in fluid connection with the capillaries 210. A second electrode plate 225 is associated with the second dielectric 215. Although two capillaries 210 are shown in Figure 2a, the reactor 200 may be designed, as desired, so that the first dielectric 205 includes one or more capillaries. The first and second dielectrics 205, 215 may be the same or different. As shown in Figure 2a, the second electrode 225 is a plate, however, any other shape including a segmented electrode similar to that of the first electrode 220 may be used. Moreover, the second electrode and second dielectric may be eliminated altogether.

A first electrode 220 is associated with the first dielectric 205. In a preferred embodiment, first electrode 220 is a plurality of electrode segments in fluid communication with respective capillaries 210. In the example shown in Figure 2a, the electrode segments are in the shape of a hollow cylinder, ring or washer that has been inserted partially into the respective capillary 210. In an alternative embodiment, the first electrode 220 may be disposed above, substantially flush with the first dielectric 205, or extend any desired depth into the capillary 210. A power supply 230 is connected to both the first and second electrodes 220, 225.

Because the electrode segments of the first electrode 220 shown in Figure 2a are hollow, an organic based reagent may be passed or injected therethrough and into the capillaries 210 defined in the first dielectric 205. A weakly ionized gas is created in the capillaries 210 and the channel 235 upon the application of a voltage from the power supply 230 to the first and second electrodes 220, 225, respectively. Introduction of the organic based reagent into the weakly ionized gas produces chemically active species in the capillaries 210 and/or channel 235 which promote chemical reactions that facilitate processes such as the destruction of contaminants and sterilization.

In operation, the object of treatment 5 (typically a contaminated fluid to be sterilized) is preferably passed along with the organic based reagent through the openings in the electrode segments of the first electrode 220 and into the respective capillaries 210 where weakly ionized gas is generated. The presence of the organic based reagent in the presence of the weakly ionized gas increases the concentration of chemically active species being produced. It is in the capillaries 210 themselves that the highest concentration of chemically active species is generated. Accordingly, the most efficient sterilization is realized by passage of the contaminated object to be treated through this region of maximum concentration of these generated ion, free radicals and active species (e.g., in the capillaries themselves). In addition, or alternatively, the object to be treated 5' (typically a contaminated solid object) may be passed through the channel 235 or placed proximate the end of the capillaries from which the weakly ionized gas after glow containing generated chemically active species is emitted (opposite placement of the first electrode). Due to the relatively short life span of the sterilizing active species after being generated (e.g., sterilizing active species having life spans in the range of several microseconds are thus able to travel only several millimeters prior to deactivation) decontamination of the object to be treated 5, 5' is substantially less effective when positioned in the channel 235 compared to passage of the object to be treated through the opening in the first electrodes 220 and capillaries 220 where the highest concentration of the chemically active species is found.

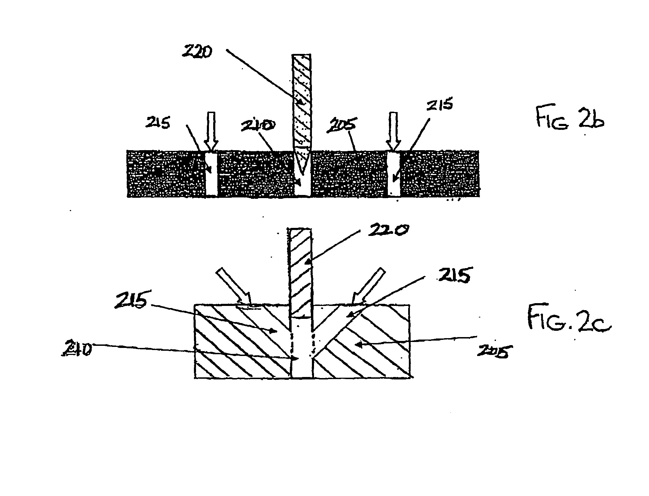

The capillary discharge reactor shown in Figure 2a may be modified to include auxiliary channels defined in the first dielectric 205. This configuration is particularly suited for those applications in which a solid or non-porous first electrode 220 is used, but does not preclude its use with a hollow or porous first electrode. Figure 2b shows a capillary discharge reactor having a single capillary 210 with a solid pin shaped annular electrode 220 terminating in a point inserted partially therein. An auxiliary channel 215 is defined through the first dielectric 205 on either side of the capillary 210. In the embodiment shown in Figure 2b, the auxiliary channels 215 are not in fluid communication with the capillary 210. An alternative configuration is shown in Figure 2c, wherein a blunt end solid pin shaped annular electrode 220 is partially inserted into the capillary 210 which is in fluid communication with auxiliary channels 215. In either configuration, the number, shape, dimension or angle of orientation of each capillary and/or auxiliary channel may be modified, as desired. In either of the embodiments shown in Figures 2b and 2c, the organic based reagent may be introduced into the auxiliary channels 215 to stabilize weakly ionized gas or increase the concentration of generated chemically active species for improving the chemical reactions. If a hollow or porous first electrode is used the organic based reagent may be injected therethrough and/or into the capillary 210 in addition to, or instead of, being injected into the auxiliary channels 215. The object to be treated 5 (typically a contaminated fluid) may be introduced along with the organic based reagent through: the first electrode 220 (if hollow or porous) and the capillary 210 and/or the auxiliary channel 215 defined in the first dielectric.

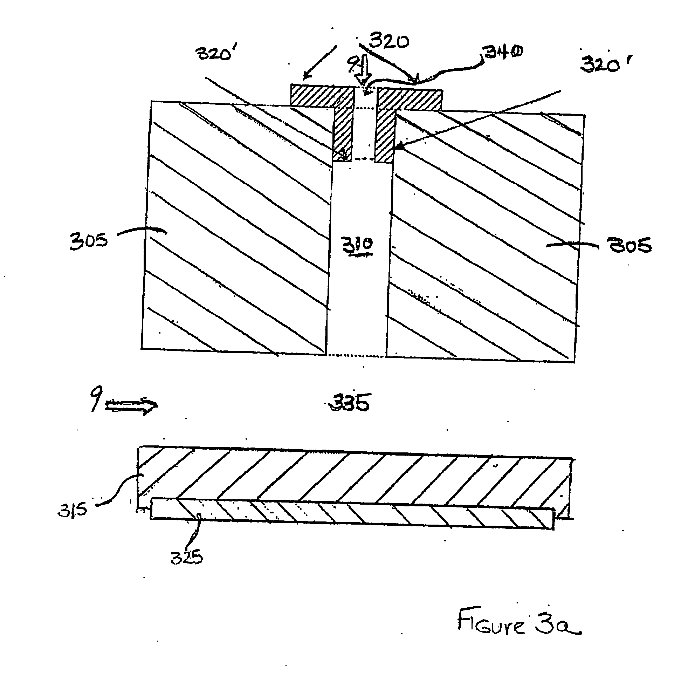

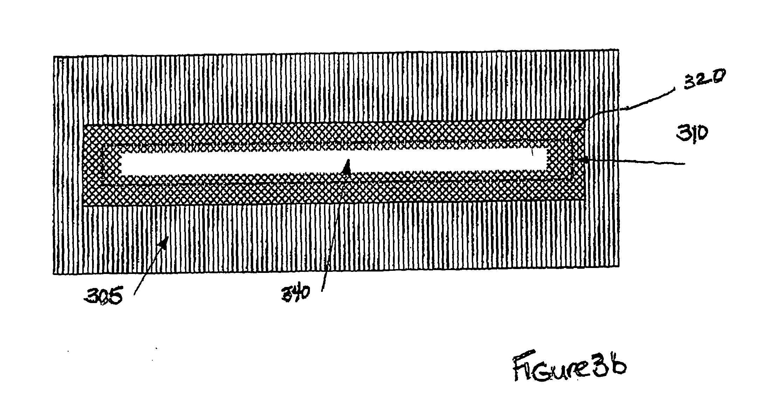

As an alternative to the capillary discharge reactor configuration, a slot discharge reactor may be employed, as disclosed in U.S. Patent Application Serial No. 10/371,243, filed February 19, 2003, having a common assignee with that of the present invention. A partial longitudinal cross-sectional view of an exemplary slot discharge reactor is shown in Figure 3a, while a top view is shown in Figure 3b. One or more slots 310 are defined in the first dielectric 305. A "slot" is defined as an aperture or opening whose length is greater than its width and whose maximum length is limited only by the dimensions of the dielectric in which it is defined. For example, the slot may have a width of about 1/32 inch and about 16 inches in length (i.e., a ratio of length to width of 512:1) in the first dielectric plate. A one inch border may be retained between the end of the slot and the end of the dielectric plate. The slots may be arranged so that adjacent slots are separate from one another by about 1/8 inch. This example and its dimensions are not intended to limit the scope of the present invention. In a preferred embodiment, the length to width ratio is at least approximately 10:1. Greater length to width ratios are possible even ratios larger in size by orders of magnitude such as at least approximately 100:1 or at least approximately 1000:1. In another embodiment, the length to width ratio is approximately 10:1 to approximately 100:1, 1,000:1, or 10,000:1 or approximately 100:1 to approximately 1000:1 or 10,000:1. These are but a few examples, other length to width slot ratios are contemplated and within the intended scope of the invention.

In the capillary discharge reactor shown in Figures 2a-2c, the capillary dimensions are substantially equal to one another in the x-y plane defined as the lateral plane substantially transverse to the longitudinal or axial direction of the capillary. Thus, there is little, if any, difference in which radial direction the electron drifts as it travels down the capillary since the probability of quenching (colliding with a wall) prior to emerging from the capillary is substantially equivalent in both the x- and y-directions. In the case of a slot, the length, e.g., the y-direction, is substantially greater than that of its width, e.g., the x-direction. Preferably, the length to width ratio of the slot is at least approximately 10 to 1. As a result of this substantially greater length to width ratio, the probability that an electron while drifting in a z-direction down the slot through the dielectric will collide or interact with the wall before emerging from the slot is significantly reduced. This reduction in the probability of wall collisions means less quenching and thus a higher plasma density per unit area of slot cross-section as compared to plasma density per unit area of a capillary discharge configuration. Despite the decreased quenching the slot still adequately suppresses glow-to-arc transition.

Regardless of the number of capillaries arranged in series one after the other packed together, the slot configuration in accordance with the present invention is able to generate a greater volume of weakly ionized gas relative to that of the capillary discharge design. Thus, a significantly greater area and volume of weakly ionized gas may be realized using the slot configuration in comparison to that of the capillary configuration. As a result of the increased volume of weakly ionized gas generated, a relatively large volume of fluid/gas to be treated may be exposed to the weakly ionized gas. Moreover this slot configuration may be readily and inexpensively manufactured.

Figures 3a and 3b show a partial longitudinal cross-sectional view and top view, respectively, of an exemplary single electrode segment 320 and an associated slot 310 in the first dielectric 305. Although only a single slot and associated electrode segment is shown, the same electrode segment structure and arrangement may be used for a reactor having multiple slots. Figure 3a is a partial cross-sectional view of an exemplary rectangular shaped rim electrode segment 320 having a T-shaped cross-section with a portion thereof 320' inserted partially into a respective slot 310 defined in a first dielectric 305. Shoulder components insure that the first electrode 320 is inserted substantially uniformly and securely in the slot 310, however the shoulder regions 325 are not essential and the first electrode may be shaped as a cylindrical hollow tube. Alternative configurations of the shape of the first electrode 320 and its relationship relative to the slot 310 may be modified, as desired. Since rectangular shaped rim electrode segment 320 is open or hollow an organic based reagent and/or the object to be treated 5 may be passed through the opening 340 of the first electrode 320 itself and into the slot 310 of the first dielectric 305. Under such conditions, treatment of the object to be treated 5 by exposure to the plasma may occur in the slot 310 where the highest concentration of chemically active species exists. Alternatively, or in addition thereto, the organic based reagent and/or the object to be treated 5' may be passed through or disposed in the channel 335 between the first and second dielectrics 305, 315, respectively.

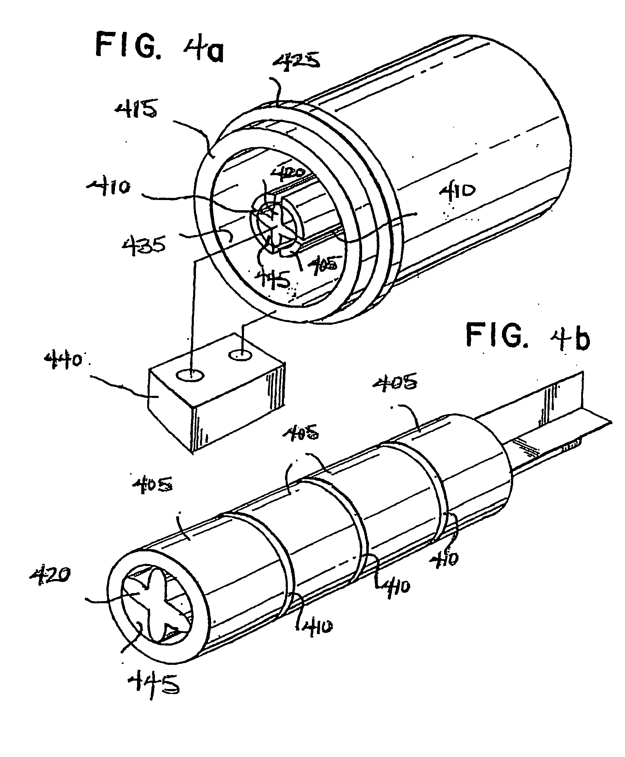

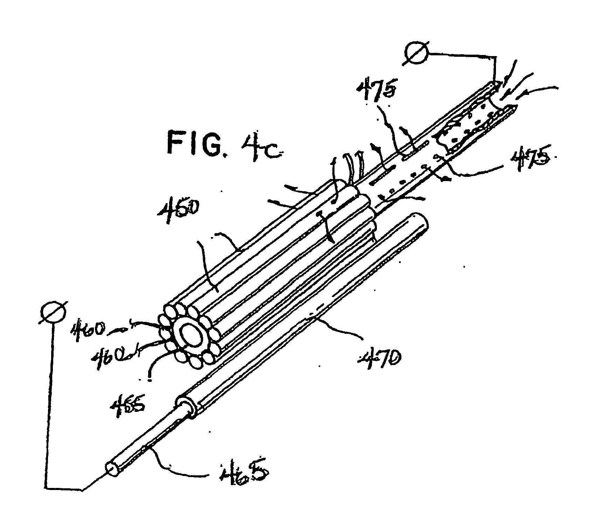

Still another configuration of the weakly ionized gas reactor is a slit discharge reactor, as described in U.S. Patent Application Serial No. 10/287,772, filed November 2, 2002, and assigned to the same assignees of the present invention. Figure 4a is a perspective view of a first embodiment of an annular slit discharge reactor. The first or primary dielectric annular tube 405 is longitudinally divided into four radial sections with adjacent sections separated a predetermined distance from one another to form a slit 410 therebetween disposed in a longitudinal axial direction. Primary or first electrode 420 comprises four blades disposed to form a star with each blade extending longitudinally through the first dielectric annular tube 405 and disposed proximate and in fluid communication with a corresponding slit 410. A receiving or second annular electrode 425 encloses the first dielectric 405 with a second annular dielectric 415 disposed between the first dielectric 405 and second annular electrode 425. The first electrode 420 and second annular electrode 425 are connected to a power source 440. A channel 435 is formed between the first and second dielectrics 405, 415, respectively, to which an organic based reagent and/or object to be treated is received. Alternatively, or in addition thereto, an organic based reagent and/or object to be treated may be received through a passageway 445 between the first electrode 420 and inner first dielectric 405. Figure 4a shows the first dielectric 405 divided longitudinally into four radial sections, however, it is contemplated and within the intended scope of the invention to divide the first dielectric into any number of two or more sections, that may, but need not necessarily, be of equal size, whereby the first electrode 420 will preferably be configured with an equal number of blades as slits 410 in the first dielectric.

Figure 4b shows a modification of the configuration of the inner first dielectric of Figure 4a - instead of being divided so as to form longitudinal slits therein (Figure 4a), the first dielectric 405 may be divided laterally into sections thereby separating the inner dielectric cylindrical tube into a series of rings 405. As shown in Figure 4b, the first dielectric 405 is divided laterally into four sections or rings with adjacent sections separated a predetermined distance to form a slit 410 therebetween. In keeping with the invention, the inner first dielectric 405 may be divided radially into any number of rings with the size of the rings being either the same or different. In still another embodiment, the slit 410 may be defined as a spiral through the cylindrical shaped inner first dielectric 405 with a wire serving as the first electrode disposed substantially aligned or crossing over the spiral slit.

Figure 4c shows yet another embodiment of the slit discharge reactor wherein a plurality of first dielectric rods 450 are disposed radially about the outer perimeter of an inner cylindrical tube 455, preferably having a hollow center to allow the passage of an organic based reagent and/or object to be treated. Twelve rods are disposed about the perimeter of the inner cylindrical tube 455 shown in Figure 4c, but the number of rods may be varied, as desired. The inner cylindrical tube 455 may be made from a conductive or a dielectric material. Dielectric rods 450 are arranged with a separation between adjacent rods to form slits that allow the passage of the organic based reagent and/or an object to be treated to dispense radially outward therefrom. In a preferred embodiment, the slits formed between adjacent dielectric rods have a width less than or equal to approximately 1 mm to obtain the desired choking effect that substantially reduces if not totally eliminates glow-to-arc transitions. In the event that the inner cylindrical tube 455 is made of a dielectric material, conductive wires or rods 460 may be inserted into the slits between adjacent dielectric rods 450 to serve as an electrode. A receiving or second annular cylindrical electrode 465, preferably enclosed by a receiving or second annular dielectric 470, is disposed proximate the dielectric rods 450 and a voltage differential is applied to the inner cylindrical electrode tube and receiving electrode 455, 465. If an AC or RF power source is used then the receiving or second electrode 465 is enclosed in a secondary dielectric layer 470 or immersed in a non-conductive liquid. On the other hand, if a DC source is used the second dielectric is not employed and the receiving or second electrode 465 may be immersed in a conducting liquid. Apertures 475 of any desired shape, arrangement and number, are defined in the first electrode 455 to permit the passage of the organic based reagent and/or object to be treated received in the inner hollow channel of the first electrode 455.

The aforementioned weak ionized gas generation reactor configurations described above all have either perforations defined in or formed between components of the dielectric. It is, however, contemplated and within the intended scope of the present invention to use an organic based reagent with reactors that do not have perforations in the dielectric such as conventional corona or barrier discharge reactors.

Furthermore, the reactor need not necessarily employ a dielectric and in such situations the organic based reagent may be passed through perforations defined in or formed between segments of the electrode. In short, the organic based reagent in accordance with the present invention is to be energized by the weakly ionized gas. Such is the case when the organic based reagent is injected, received or passed through the dielectric and electrode perforation. It is also the case when the organic based reagent is injected or mixed proximate the after glow of the weakly ionized gas. This simply means that the organic based reagent must be in the presence of the weakly ionized gas generated so as to participate in the formation of chemically active species.

The herein described present inventive system and method may be used to treat, destroy, kill, remove, purify or decontaminate undesirable chemical and/or biological agents on any object to be treated, regardless of whether it is a solid, liquid, gas, vapor or any combination thereof. Sterilization is but one particular application of the present inventive method and system for the destruction of microbiological agents. Several particular applications of the sterilization system and method in accordance with the present invention are shown and described but are not exhaustive nor intended to limit the scope of application. One noteworthy application is use of the present inventive system and method for use in cleaning or treating of filter media after being used. A weakly ionized gas generating reactor is placed upstream relative to a filter so as to simultaneously capture and destroy biological particulate matter such as spores and bacteria. The system is also suitable for capturing and destroying of chemical agents in aerosol and gaseous forms. The spore/bacteria laden air stream is filtered though a filter for capturing spores and bacteria on the surface. In turn, the filter is treated at least once, preferably continuously or periodically, by exposure to the after glow of generated chemically active species so as to destroy the spores. Since relatively large residence times of the spores on the surface can be realized this ensures a relatively high kill rate without having to reduce the air flow rate. A HEPA filter is preferably used since it is approximately 99.97% effective down to a particle size of approximately 0.3 microns. Anthrax spores, for example, have a diameter approximately 3 micron. Weaponized anthrax particulates are in the order of approximately 1-3 microns. Therefore, substantially all of these particles will be trapped in the outer surface of the filter where the desired chemical reactions from the chemically active species generated by the organic based reagent and weakly ionized gas will have a substantial effect.

All references, publications, pending and issued patents are herein each incorporated by reference in their entirety. This application also incorporates by reference U.S. Patent Application Serial No. 09/738,923, filed December 15, 2000; U.S. Provisional Application No. 60/171,198, filed December 15, 1999; U.S. Provisional Application No. 60/171,324, filed December 21, 1999; U.S. Patent Application Serial No. 10/287,772, filed November 2, 2002; U.S. Provisional Application No. 60/336,866, filed November 2, 2001; U.S. Patent Application Serial No. 10/371,243, filed February 19, 2003; U.S. Provisional Application No. 60/358,340, filed February 19, 2002; U.S. Patent Application Serial No. 10/287,771, filed November 4, 2002; and U.S. Provisional Application No. 60/336,868, filed November 2, 2001.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the spirit and scope of the invention. For example, it is expressly intended that all combinations of those elements and/or steps which perform substantially the same function, in substantially the same way, to achieve the same results are within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

The following embodiments are preferred embodiments of the present application:
1. A method for increasing concentration of chemically active species generated by weakly ionized gas used to treat an object, said method comprising the steps of:
   generating the weakly ionized gas using a reactor; and
   introducing an organic based reagent in the presence of the weakly ionized gas to increase production of the chemically active species, wherein the organic based reagent includes an organic additive and an oxidizer.
2. The method according to embodiment 1, wherein the organic additive and the oxidizer are mixed together to form the organic based reagent.
3. The method according to embodiment 2, wherein the organic additive is one of ethyl alcohol or ethylene, while the oxidizer is one of oxygen or air.
4. The method according to embodiment 1, wherein the introducing step comprises injecting the organic additive into the weakly ionized gas in the presence of air that serves as the oxidizer.
5. The method according to embodiment 1, wherein the organic based reagent is an organic additive containing an oxygen component that serves as the oxidizer.
6. The method according to embodiment 5, wherein the introducing step further comprises injecting a supplemental oxidizer into the weakly ionized gas.
7. The method according to embodiment 1, wherein the chemically active species include peroxy (RO₂)·, alkoxy (RO)·, acyl peroxyacyl (RC(O)OO)-, hydroperoxides (ROOH), peroxynitrates (RO₂NO₂), organic nitrates (RONO₂), peroxyacids (RC(O)OOH), carboxylic acids (RC(O)OH) and peroxyacyl nitrates (RC(O)O₂NO).
8. The method according to embodiment 1, wherein the reactor comprises a dielectric with an aperture defined therein and an electrode disposed in fluid communication with the aperture, the organic based reagent being introduced through the aperture and the electrode so as to interact within the aperture with the weakly ionized gas so as to generate the chemically active species.
9. The method according to embodiment 8, wherein the exposing step comprises passing the object to be treated through the aperture and electrode so as to be subject to the chemically active species generated therein.
10. The method according to embodiment 1, wherein the object to be treated is one of a solid, liquid or a gas.
11. The method according to embodiment 1, wherein said method is used for sterilization of the object to be treated to destroy at least one of chemical or biological contaminants.
12. The method according to embodiment 1, further comprising exposing the object to be treated to the generated chemically active species.
13. A system for increasing concentration of chemically active species generated by weakly ionized gas used to treat an object, the system comprising:
   a reactor for generating the weakly ionized gas, the reactor receiving an organic based reagent; and
   a source of organic based reagent, the reactor being adapted to receive the organic based reagent in the presence of the weakly ionized gas to increase the production of chemically active species; the organic based reagent includes an organic additive and an oxidizer.
14. The system according to embodiment 13, wherein the organic based reagent is a mixture of the organic additive and the oxidizer.
   22
15. The system according to embodiment 14, wherein the organic additive is one of ethyl alcohol or ethylene, while the oxidizer is one of oxygen or air.
16. The system according to embodiment 13, wherein the organic additive is received in the weakly ionized gas in the presence of air that serves as the oxidizer.
17. The system according to embodiment 13, wherein the organic based reagent is an organic additive containing an oxygen component that serves as the oxidizer.
18. The system according to embodiment 17, wherein the organic based reagent further includes a supplemental oxidizer disposed so as to be received into the weakly ionized gas.
19. The system according to embodiment 13, wherein the chemically active species include peroxy (RO₂)·, alkoxy (RO)·, acyl peroxyacyl (RC(O)OO)·, hydroperoxides (ROOH), peroxynitrates (RO₂NO₂), organic nitrates (RONO₂), peroxyacids (RC(O)OOH), carboxylic acids (RC(O)OH) and peroxyacyl nitrates (RC(O)O₂NO).
20. The system according to embodiment 13, wherein the reactor comprises a dielectric with an aperture defined therein and an electrode disposed in fluid communication with the aperture, the organic based reagent being adapted to be received through the aperture and the electrode so as to interact within the aperture with the weakly ionized gas so as to generate the chemically active species.
21. The system according to embodiment 20, the aperture and electrode are adapted to receive the object to be treated therethrough so as to be subject to the chemically active species generated therein.
22. The system according to embodiment 13, wherein the object to be treated is one of a solid, liquid or a gas.
23. The system according to embodiment 13, wherein the system is used for sterilization of the object to be treated to destroy at least one of chemical or biological contaminants.
24. The system according to embodiment 13, wherein the reactor is disposed relative to the object to be treated so that the generated chemically active species interact with the object to be treated.

## Claims

1. A method for increasing concentration of chemically active species generated by weakly ionized gas used to treat an object, said method comprising the steps of:
generating the weakly ionized gas using a reactor; and
introducing an organic based reagent in the presence of the weakly ionized gas to increase production of the chemically active species, wherein the organic based reagent includes ethylene and an oxidizer.

2. The method according to claim 1, wherein the ethylene and the oxidizer are mixed together to form the organic based reagent, prior to being introduced to the weakly ionized gas.

3. The method according to claim 2, wherein the oxidizer is one of oxygen or air.

4. The method according to claim 1, wherein the introducing step comprises the step of injecting the ethylene into the weakly ionized gas in the presence of air.

5. The method according to claim 1, wherein the organic based reagent contains an oxygen component that serves as the oxidizer.

6. The method according to claim 1, wherein the introducing step further comprises the step of injecting a supplemental oxidizer into the weakly ionized gas.

7. The method according to claim 1, wherein the chemically active species include peroxy (RO₂)·, alkoxy (RO)·, acyl peroxyacyl (RC(O)OO)·, hydroperoxides (ROOH), peroxynitrates (RO₂NO₂), organic nitrates (RONO₂), peroxyacids (RC(O)OOH), carboxylic acids (RC(O)OH) and peroxyacyl nitrates (RC(O)O₂NO).

8. The method according to claim 1, wherein the object to be treated is one of a solid, liquid or a gas.

9. The method according to claim 1, wherein said method is used for sterilization of the object to be treated to destroy at least one of chemical or biological contaminants.

10. The method according to claim 1, further comprising exposing the object to be treated to the generated chemically active species.

11. The method according to claim 1, wherein the reactor comprises a dielectric with an aperture defined therein and an electrode disposed in fluid communication with the aperture, the organic based reagent being introduced through the aperture and the electrode so as to interact within the aperture with the weakly ionized gas so as to generate the chemically active species.

12. The method according to claim 11, further comprising the step of exposing the object to be treated, to the chemically active species, by passing the object through the aperture and electrode so as to subject the object to the chemically active species generated therein.

13. The method according to claim 1, wherein the weakly ionized gas is generated at atmospheric pressure.

14. The method according to claim 1, wherein the reactor comprises a porous electrode.

15. A system for increasing concentration of chemically active species generated by weakly ionized gas used to treat an object, the system comprising:
a reactor for generating the weakly ionized gas; and
a source of organic based reagent, the reactor being adapted to receive the organic based reagent in the presence of the weakly ionized gas to increase the production of chemically active species; the organic based reagent includes ethylene and an oxidizer.

16. The system according to claim 15, wherein the organic based reagent is a mixture of the ethylene and the oxidizer.

17. The system according to claim 16, wherein the oxidizer is one of oxygen or air.

18. The system according to claim 15, wherein the ethylene is received in the weakly ionized gas in the presence of air.

19. The system according to claim 15, wherein the organic based reagent is the ethylene containing an oxygen component.

20. The system according to claim 19, wherein the organic based reagent further includes a supplemental oxidizer disposed so as to be received into the weakly ionized gas.

21. The system according to claim 15, wherein the chemically active species include peroxy (RO₂)·, alkoxy (RO)·, acyl peroxyacyl (RC(O)OO)·, hydroperoxides (ROOH), peroxynitrates (RO₂NO₂), organic nitrates (RONO₂), peroxyacids (RC(O)OOH), carboxylic acids (RC(O)OH) and peroxyacyl nitrates (RC(O)O₂NO).

22. The system according to claim 15, wherein the reactor is adapted to receive the object to be treated so as to be subject to the chemically active species generated therein.

23. The system according to claim 15, wherein the object to be treated is one of a solid, liquid or a gas.

24. The system according to claim 15, wherein the system is used for sterilization of the object to be treated to destroy at least one of chemical or biological contaminants.

25. The system according to claim 15, wherein the reactor is disposed relative to the object to be treated so that the generated chemically active species interact with the object to be treated.

26. The system according to claim 15, wherein the reactor comprises a dielectric with an aperture defined therein and an electrode disposed in fluid communication with the aperture, the organic based reagent being adapted to be received through the aperture and the electrode so as to interact within the aperture with the weakly ionized gas so as to generate the chemically active species.

27. The system according to claim 26, wherein the aperture and electrode are adapted to receive the object to be treated therethrough so as to be subject to the chemically active species generated therein.

28. The system according to claim 15, wherein the reactor comprises a porous electrode.

29. A method of sterilizing an object using a plasma reactor comprising the steps of (a) generating a plasma from a mixture of ethylene and an oxidizer to produce a chemically active species, and (b) exposing the object to the chemically active species.

30. The method of claim 29, wherein the object is exposed to the chemically active species emitted from the reactor.

31. The method of claim 29, wherein the plasma is generated at atmospheric pressure.

32. The method of claim 30, wherein the plasma is generated at atmospheric pressure.

33. A method for producing chemically active species used to treat an object comprising the steps of:
introducing an organic based reagent into a gas discharge plasma reactor, wherein the organic based reagent comprises ethylene and an oxidizer; and
generating chemically active species from the organic based reagent with the gas discharge plasma reactor.

34. The method according to claim 33, wherein the ethylene and the oxidizer are mixed together to form the organic based reagent, prior to being introduced to the gas discharge plasma reactor.

35. The method according to claim 33, wherein the oxidizer comprises at least one of oxygen or air.

36. The method according to claim 33, wherein the oxidizer is oxygen.

37. The method according to claim 33, wherein the oxidizer is air.

38. The method according to claim 33, wherein the introducing step comprises the step of injecting the ethylene into the gas discharge plasma reactor in the presence of air.

39. The method according to claim 33, wherein the introducing step further comprises the step of injecting a supplemental oxidizer into the gas discharge plasma reactor.

40. The method according to claim 33, wherein the object to be treated is one of a solid, liquid, or gas.

41. The method according to claim 33, further comprising exposing the object to the chemically active species.

42. The method according to claim 41, wherein the object is exposed to the chemically active species for a sufficient time to destroy chemical or biological contaminants.

43. The method according to claim 41, wherein the object is exposed to the chemically active species for a sufficient time to sterilize the object.

44. The method according to claim 33, wherein the gas discharge plasma reactor comprises a dielectric with an aperture defined therein and an electrode disposed in fluid communication with the aperture, the organic based reagent being introduced through the aperture and the electrode to generate the chemically active species.

45. The method according to claim 44, further comprising the step of exposing the object to be treated, to the chemically active species, by passing the object through the aperture and electrode so as to subject the object to the chemically active species generated therein.

46. The method according to claim 33, wherein the chemically active species is generated at atmospheric pressure.

47. The method according to claim 33, wherein the gas discharge plasma reactor comprises a porous electrode.

48. A system for producing chemically active species used to treat an object comprising:
a gas discharge plasma reactor; and
a source of an organic based reagent comprising ethylene and an oxidizer, wherein the reactor is adapted to receive the organic based reagent to produce chemically active species therefrom.

49. The system according to claim 48, wherein the ethylene and the oxidizer are mixed together to form the organic based reagent, prior to being introduced to the gas discharge plasma reactor.

50. The system according to claim 48, wherein the oxidizer comprises at least one of oxygen or air.

51. The system according to claim 48, wherein the oxidizer is oxygen.

52. The system according to claim 48, wherein the oxidizer is air.

53. The system according to claim 48, wherein the introducing step comprises the step of injecting the ethylene into the gas discharge plasma reactor in the presence of air.

54. The system according to claim 48, wherein the introducing step further comprises the step of injecting a supplemental oxidizer into the gas discharge plasma reactor.

55. The system according to claim 48, wherein the object to be treated is one of a solid, liquid, or gas.

56. The system according to claim 48, further comprising exposing the object to the chemically active species.

57. The system according to claim 56, wherein the object is exposed to the chemically active species for a sufficient time to destroy chemical or biological contaminants.

58. The system according to claim 56, wherein the object is exposed to the chemically active species for a sufficient time to sterilize the object.

59. The system according to claim 48, wherein the gas discharge plasma reactor comprises a dielectric with an aperture defined therein and an electrode disposed in fluid communication with the aperture, the organic based reagent being introduced through the aperture and the electrode to generate the chemically active species.

60. The system according to claim 59, further comprising the step of exposing the object to be treated, to the chemically active species, by passing the object through the aperture and electrode so as to subject the object to the chemically active species generated therein.

61. The system according to claim 48, wherein the chemically active species is generated at atmospheric pressure.

62. The system according to claim 48, wherein the gas discharge plasma reactor comprises a porous electrode.
